# EUROPEAN PATENT SPECIFICATION

(11) **EP 0 554 602 B1**
(45) Date of publication and mention of the grant of the patent: **18.12.1996**
(21) Application number: 92305986.9
(22) Date of filing: 29.06.1992
(51) Int. Cl.: A61B 17/12

(54) **Compressive hemostatic belt**
Hämostatischer Druckgürtel
Ceinture hémostatique à pression

(30) Priority: 06.02.1992 JP 20793/92; 22.04.1992 JP 102850/92; 22.04.1992 JP 102851/92
(43) Date of publication of application: 11.08.1993
(73) Proprietor: SUMITOMO RUBBER INDUSTRIES LIMITED, Kobe-shi Hyogo-ken (JP)
(72) Inventor: Hori, Shinichi, Sakai-shi, Osaka-fu (JP); Kawasaki, Atsuko, Akashi-shi, Hyogo-ken (JP); Nakashita, Takefumi, Kobe-shi, Hyogo-ken (JP); Inui, Yoshiharu, Takarazuka-shi, Hyogo-ken (JP); Sakaki, Toshiaki, Kakogawa-shi, Hyogo-ken (JP); Miki, Miyo, Kobe-shi, Hyogo-ken (JP)
(74) Representative: Hillier, Peter

(56) References cited:
- EP-A- 0 514 026
- DE-A- 3 333 311
- DE-A- 4 012 974
- DE-C- 56 336
- DE-C- 571 744
- FR-A- 910 340
- GB-A- 1 206 605
- US-A- 3 171 410
- US-A- 3 633 567
- US-A- 3 670 735
- US-A- 4 275 718

## Description

### BACKGROUND OF THE INVENTION

### Field of the Invention

The present invention relates to a compressive hemostatic belt used to stop bleeding from a catheter insertion wound upon completion of an arterial catheter examination.

### Prior Art

Recently, arterial catheter examinations have been made for contrast medium-using diagnosis of hearts or cerebral blood vessels. Cardiac catheter examinations are made by surgical operation in a few cases but in most cases by the so-called catheter puncture method where a catheter is moved from the femoral artery or vein in the inguinal region to the heart.

In this examination method, a contrast medium or various medicines are injected through the catheter puncturing the femoral artery or vein in the inguinal region or various preoperative and postoperative examinations are conducted. In this connection, there is a need to compress the catheter insertion wound for a relatively long time in order to stop bleeding from the catheter insertion wound owing to extraction of the catheter from the femoral artery or vein in the inguinal region.

As for such compressive hemostatic method for catheter insertion wounds, it has been common practice for a doctor or nurse to manually compress a catheter insertion wound for about 15 minutes, apply gauze to the catheter insertion wound, apply 3 or 4, 70-cm long 5-cm wide fabric adhesive plasters over said gauze to compress said catheter insertion wound from above said gauze, placing a sand bag having a controlled weight of 500 - 1000 g, and fix said sand bag against moving by said fabric adhesive plaster, such fixed state being maintained for 12 - 24 hours.

However, in the conventional method described above, the use of adhesive plaster as means for fixing the gauze applied to the catheter insertion wound and also fixing the sand bag placed thereon after extraction of the catheter causes such drawbacks to the patient as a stiffening feel, pain and itch and favors the development of dermatitis and vesicular exanthema.

The sand bag tends to slip off when the patient lying on his back tilts his body even slightly, and such deviation of the sand bag results in the sand bag deviating from the compressed area, making the hemostatic effect imperfect, leading to other drawbacks, such as ecchymoma.

On the other hand, in order to solve said problems, Japanese Patent Application Disclosure No. 92746/1985 or Japanese Patent Application Disclosure No. 198139/1985 suggests a compressive hemostatic belt formed of stretch fabric as means for compressive hemostatic means for catheter insertion wounds to replace the adhesive plaster and sand bag.

Such compressive hemostatic belt, however, is constructed to make it difficult for a user to wrap it and to visually ascertain the level of its compressive force on the catheter insertion wound and makes it necessary for him to rewrap it when adjusting the compressive force; thus, the loading state of the belt is unstable.

Further, since the belt uses an expensive stretch textile fabric, its production cost is very high, making it difficult to throw it away after use when there is a hygienic problem of causing infectious diseases due to adhesion of blood. Therefore, to prevent such hemoinfectious diseases, after each use of such compressive hemostatic belt, it has to be washed and sterilized, imposing limitations not only from a hygienic standpoint but also from the standpoint of enhancing labor saving for nurses.

### SUMMARY OF THE INVENTION

In a later published, and therefore falling under the terms of Article 54(3) EPC, prior art document, EP-A-0514026, a compressive hemostatic belt is described having a balloon expansible by being filled with a fluid attached to a strip in the form of a non stretch or low-stretch textile fabric.

DE 4012974 discloses a hemostatic belt in which two cuff bags each have an inlay which allows inward expansion only and DE 571 744 discloses a hemostatic belt having air cushions of which the upper region is rigid and the lower portion is elastic.

In order to solve the above described problems the present invention provides a compressive hemostatic belt comprising a balloon (5) inflatable by being filled with fluid and mounted by mounting means on a specific position of a non-elastic or low-elastic strip (1), characterised in that a non-elastic reinforcing member (11) is integrally applied on a side of said balloon (5) confronting said strip (1). Said strip may be made of non-elastic or low-elastic fibre cloth.

The invention also provides a compressive hemostatic belt comprising a balloon (5) inflatable by being filled with fluid and mounted by mounting means on a specific position of a non-elastic or low-elastic strip (1), characterised in that the balloon (5) is thick at the side confronting said strip (1), and thin at the other side confronting the stopping area. By filling the balloon with proper fluid, the balloon is inflated to pressure an area where bleeding is to be stopped.

Said mounting means may be a pocket (3) formed at a specific position of said strip (1), being capable of incorporating the balloon (5) inside thereof. A side of said pocket (3) confronting the area where bleeding is to be stopped is made of an expandable sheet member. Also, a rubber-made balloon (5) inflated by fluid may be contained in the pocket (3) of said strip (1).

A fluid feed pump (8) and a pressure gauge (9) may be detachably connected to said balloon (5) by way of a check valve (6). Therefore, while observing the pressure gauge, the pump may be manipulated to fill the balloon with fluid to pressure the area where bleeding is to be stopped.

In an embodiment a portion of said strip (1) at least confronting the balloon (5) is formed of a non-elastic reinforcing member made of material stronger than that of the other portions. As a result, without using any hard material, the balloon can be inflated only to the area where bleeding is to be stopped while preventing inflation to the side opposite to that area.

In an embodiment, plural non-elastic reinforcing fibre cloths are overlaid at least in the portion of said strip (1) confronting the balloon (5).

The confronting surface of the pocket (3) of said strip (1) against the stopping area may be formed of an elastic fibre cloth.

According to the invention, since the strip is composed of non-elastic or low-elastic fibre cloth or the like, the development of a stiffening feeling, pain or itch can be avoided and dermatitis and bubbles are prevented. The use of the balloon which is expanded by being filled with fluid as pressure means provided a narrower rant of pressure, ensuring a hemostatic function for pressuring only a catheter insertion wound while causing almost no pressure feeling to be transmitted to other areas.

In addition, by composing a loading structure by connecting a pump and a pressure gauge through a check valve to a balloon that can be inflated by filling with fluid, any pressure desired to be applied to the catheter insertion wound may be freely adjusted and the pressure may be maintained for a long time by the check valve.

Furthermore, the hemostatic effect and fixing are achieved simultaneously by the balloon being filled with fluid. A compressive hemostatic belt is provided whose operability is satisfactory and does not require a sand bag or the like and is compact and easy to carry.

Furthermore, the constituent members excluding the pump and pressure gauge are much cheaper than conventional parts, allowing the belt to be thrown away once it has been used; thus, noteworthy effects from the standpoint of improving health control and promoting labor saving are attained.

The invention employs the balloon which is inflated largely only at the side opposite to a wounded part and not inflated so much on the outer side, requiring no hard case. The pressure means comprises only an elastic balloon, posing no problem when it is wound on the body, and enabling to provide the belt of this invention at a lower price where the disposable use of it pays.

In a preferred embodiment of the invention, moreover, as the portion of the band confronting the balloon is strengthened to prevent the balloon from inflating to the opposite side of the area where bleeding is to be stopped, requiring no hard case. The pressure means comprises only an elastic balloon, posing no problem when it is wound on the body, enabling to provide the belt of this invention at a lower price where the disposable use of it pays and causing no problem on the environmental pollution.

Embodiments of the invention will now be described by way of example only with reference to the accompanying drawings, in which:-
Fig. 1 is a front view showing an example of a compressive hemostatic belt.
Fig. 2 is a longitudinal sectional view at the pocket position of the belt shown in Figure 1.
Fig. 3 is a longitudinal sectional view at the pocket position showing the state of use of the belt shown in Figure 1.
Fig. 4 is an explanatory diagram showing a winding example of a belt.
Fig. 5 is a longitudinal sectional view showing the state of pressuring the wound with the belt of Figure 1.
Fig. 6 is a longitudinal sectional view of a balloon in a first embodiment of the invention.
Fig. 7 is a longitudinal sectional view showing the state of inflation of the balloon in the first embodiment.
Fig. 8 is a longitudinal sectional view showing the state of pressuring the wound in the first embodiment.
Fig. 9 is a longitudinal sectional view of a balloon in a second embodiment of the invention.
Fig. 10 is a longitudinal sectional view showing the state of inflation of the balloon in the second embodiment.
Fig. 11 is a longitudinal sectional view showing the state of pressuring the wound in the second embodiment.
Fig. 12 is an explanatory diagram showing a further winding example of a belt for tourniquet.

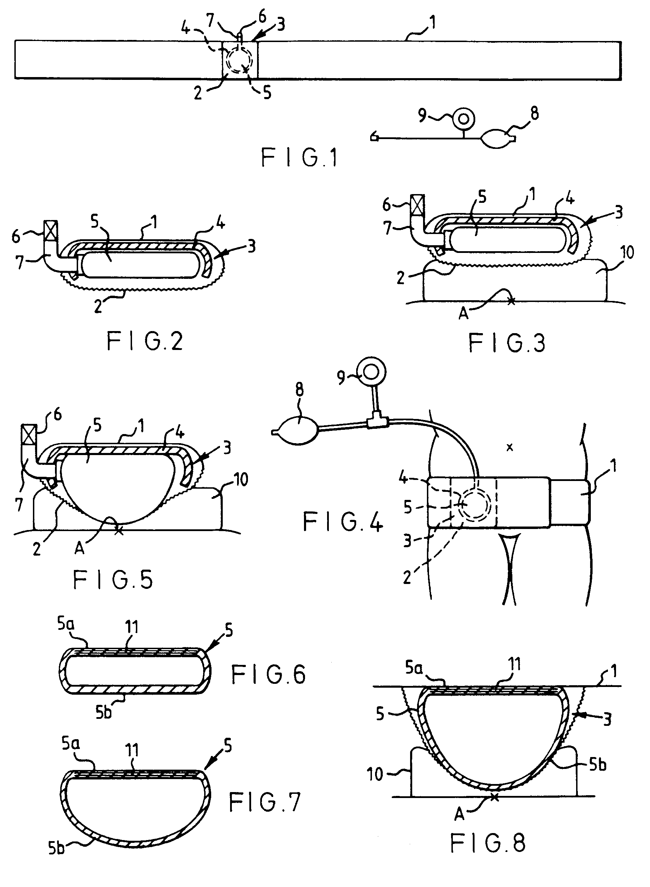

Figs. 1 and 2 show an example of a hemostatic which is not exemplary of the invention, in which numeral 1 denotes a strip composed of a non-elastic or low-elastic fiber cloth, and a pocket 3 is formed by sewing a pocket cloth 2 made of an elastic woven cloth on a specified position of the strip 1. Numeral 4 is a hard case made of a hard material such as synthetic resin formed in a cup form, and it is put in the pocket 3 of the strip 1 with the opening side directed to the pocket cloth 2. Numeral 5 is a rubber-made balloon which is inflated when filled with a proper fluid (air, nitrogen gas, carbon dioxide or other gas, or liquid including gel), and it is put in the hard case 4 with a fluid feed tube 7 possessing a check valve 6 projected outside in the pocket 3 of the strip 1. To the fluid feed tube 7 of this balloon 5, for example, a manually-operated pump 8 and a pressure gauge 9 are connected, and by manipulating the pump 8 while observing the pressure gauge 9, the balloon 5 is filled with fluid through the fluid feed tube 7, so that the balloon 5 may be inflated.

The procedure of using the belt as a tourniquet is described below.

As shown in Fig. 3, piece of gauze 10 is put on a catheter insertion wound A after removing the catheter, the pocket piece 2 of the pocket 3 containing the balloon 5 is put thereon, directed to the gauze 10 side, and the strip 1 is wound around the waist of the patient by one turn or more, and its end portion is fixed by using fixing means such as adhesive plaster to wind around the body of the patient.

When winding of the strip 1 is over in this manner, the manually-operated pump 8 and pressure gauge 9 are connected to the fluid feed tube 7 of the balloon 5, and the pump 8 is manipulated while observing the pressure gauge 9 to fill the balloon 5 with fluid, so that the balloon is inflated at the catheter insertion wound A side while restricting the inflation to the opposite side of the catheter insertion wound A by the hard case 4 as shown in Fig. 5, and the catheter insertion wound A is oppressed through the gauze 10 while fixing the strip 1 firmly to the patient.

When liquid is used as the fluid for inflating the balloon 5, as compared with gas, it hardly escapes the balloon 5 and the connected check valve 6, and if leaking, the strip 1 or other part is wet so that the escape may be known immediately, and it is easy to keep constant the force of oppression.

Thus, in the belt for tourniquet, since the catheter insertion wound A is oppressed by making use of the expansion of the balloon, only the catheter insertion wound A can be oppressed, and the sensation of oppression is hardly propagated to other portions. Still more, by manipulating the pump 8 while observing the pressure gauge 9 depending on the physique of the patient, the filling degree of the balloon with fluid is adjusted, and the force of the oppression applied to the catheter insertion wound A may be freely selected, and the hemostatic action suited to the patient may be effected, while the selected force of oppression may be maintained for a long time by the check valve 6, and it is also possible to control the oppression force automatically, if necessary, by using a computer. In addition, since the strip 1 is made of non- elastic or low- elastic fiber cloth, it is free from stickiness, pain or itchiness, and prevents dermatitis or bulla.

When the entire strip 1 is formed in a bag by using non- elastic or low-elastic fiber cloth or the like, if the pocket 3 is made loose to allow inflation of the balloon 5, the oppression force to the catheter insertion wound A by inflation of the balloon 5 is sufficient. Alternately, if the confronting side against the catheter insertion wound A of the pocket 3 accommodating the balloon 5 of the strip 1 is made of an elastic fiber, the oppression force to the catheter insertion wound A is made secure.

In the belt of figures 1 to 5, the balloon 5 is incorporated in the pocket 3 of the strip 1 through a hard case 4 for suppressing the inflation to the opposite side of the catheter insertion wound A, but, instead, the balloon 5 may be exposed and placed in the pocket 3 of the strip 1. In this case, too, the balloon 5 is inflated to the catheter insertion wound A side while suppressing the inflation to the opposite side of the catheter insert ion wound A side by the strip 1.

As shown in Fig. 4, the strip 1 is wound around at right angle to the bodily axis, but the strip 1 may also be wound in an X-form as shown in Fig. 16. That is, the strip 1 is pulled around from the inguinal part to the femoral outside part, femoral back part and femoral inside part, and from the hip joint pat to the femoral front side, and crossed in an X-form at the inguinal part, and the remaining portion is wound around the abdomen by one turn or more, and the end portion is wound around by fixing with fixing means such as nondirectional cloth fastener.

In the belt of figures 1 to 5, a fiber cloth is used as the strip 1, but instead of fiber cloth, other non-elastic or low- elastic materials may be used such as leather and film, and the pocket cloth 2 is made of elastic fiber cloth, and instead of this elastic fiber cloth, other elastic materials may be also used such as rubber.

Or the pocket may be formed by forming a part of the strip 1 in a bag shape, in which the hard case 4 and balloon 5 may be incorporated.

Since the inflation of the balloon 5 to the opposite side of the catheter insertion wound A is prevented by means of the hard case 4, when the strip 1 is wound around the body, the hard case 4 may contact with the body, which may be felt uncomfortable. Accordingly, to prevent inflation of the balloon 5 to the opposite side of the catheter insertion wound A, the invention presents first and second embodiments as described below.

In the first embodiment, the balloon 5 in the constitution of the first embodiment is structured as shown in Fig. 6. At the side of the balloon 5 confronting the strip 1, that is, at the outside 5a, a sheet of non-elastic reinforcing member 11 made of cloth, mesh, film, PET, paper or the like is inserted. The non-elastic reinforcing member 11 is integrally inserted in the outside 5a of the balloon 5 when manufacturing the balloon 5. In short, when manufacturing the balloon 5 in the latex immersion method, once the balloon die is immersed in latex, and the non- elastic reinforcing member 11 is adhered to one side for forming the outside 5a of the balloon 5 of this balloon die, and only this side of the balloon die adhered with the non- elastic reinforcing member 11 is immersed again in latex, and vulcanized, so that the non-elastic reinforcing member 11 is integrally inserted into the outside 5a of the balloon 5.

In the first embodiment, when the balloon 5 is filled with fluid, as shown in Fig. 7, the outside 5a of the balloon 5 is not inflated due to the non-elastic reinforcing member 11, while only the side confronting the catheter insertion wound A, that is, the ailing side 5b is inflated largely. Accordingly, as shown in Fig. 8, the balloon 5 is prevented from inflating to the opposite side of the catheter insertion wound A, and is inflated only to the catheter insertion wound A side, so that the catheter insertion wound A may be oppressed with a sufficient force through the gauze 10.

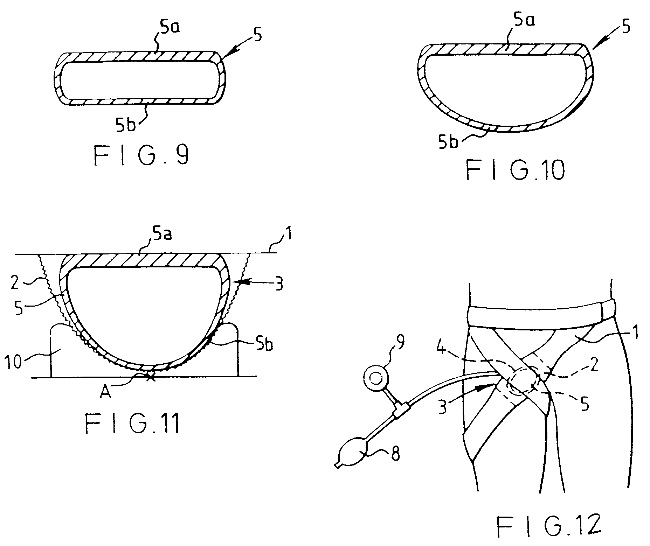

Next, in the second embodiment, the balloon 5 is structured as shown in Fig. 9. That is, the thickness of the balloon 5 is thin at the ailing side 5b, and thick at the outside 5a at a specific rate to the ailing side 5b. The thickness of the ailing side 5b is about 0.1 to 2.0 mm, and if thinner, it may lead to problems such as pinholes or aging or tearing in the course of storage or transportation, or if thicker, it is difficult to fill with fluid or the force of oppression is lowered as compared with the internal pressure. Besides, the thickness of the outside 5a is about 0.5 to 5.0 mm, and this reason is same as above, and especially in a greater thickness, it involves problems in manufacturing method or others.

In the second embodiment, if the balloon 5 is filled with fluid, the outside 5a of the balloon 5 is not inflated so much because of the great thickness as shown in Fig. 10, while the ailing side 5b is thin and is inflated largely. As a result, as shown in Fig. 11, the balloon 5 is prevented from inflating to the opposite side of the catheter insertion wound A, and it is inflated only to the catheter insertion wound A side, so that the catheter insertion wound A maybe oppressed with a sufficient force through the gauze 10.

Thus, in the first and second embodiments, without using the hard case 4 as in the hemostatic belt of Figures 1 and 2, the discomfort due to contact with the hard case 4 with the body when wound around the body experienced in the first embodiment is eliminated, and the cost for disposal may be lowered, so that it may be presented at a lower cost.

In the foregoing embodiments, fiber cloth is used as the strip 1, but instead of the fiber cloth, leather or film or other non-elastic or low-elastic material may be used, and the pocket cloth 2 is made of elastic fiber cloth, but instead of the elastic fiber cloth, rubber or other elastic material may be used, too.

## Claims

1. A compressive hemostatic belt comprising a balloon (5) inflatable by being filled with fluid and mounted by mounting means on a specific position of a non-elastic or low-elastic strip (1), characterised in that a non-elastic reinforcing member (11) is integrally applied on a side of said balloon (5) confronting said strip (1).

2. A compressive hemostatic belt comprising a balloon (5) inflatable by being filled with fluid and mounted by mounting means on a specific position of a non-elastic or low-elastic strip (1), characterised in that the balloon (5) is thick at the side confronting said strip (1), and thin at the other side confronting the stopping area.

3. A compressive hemostatic belt according to claim 1, characterised in that said strip is made of non-elastic or low-elastic fiber cloth.

4. A compressive hemostatic belt according to any of claims 1 to 3 characterised in that said mounting means is a pocket (3) formed at a specific position of said strip (1), being capable of incorporating the balloon (5) inside thereof.

5. Compressive hemostatic belt according to claim 4 characterised in that a side of said pocket (3) confronting the area where bleeding is to be stopped is made of an expandable sheet member.

6. A compressive hemostatic belt according to any of claims 1 to 5 characterised in that a fluid feed pump (8) and a pressure gauge (9) are detachably connected to said balloon (5) by way of a check valve (6).

7. A compressive hemostatic belt according to claim 1 or claim 2 characterised in that a portion of said strip (1) at least confronting the balloon (5) is formed of a non-elastic reinforcing member made of material stronger than that of the other portions.

8. A compressive hemostatic belt according to claim 1 or claim 2 characterised in that plural non-elastic reinforcing fiber cloths are overlaid at least in the portion of said strip (1) confronting the balloon (5).

9. A compressive hemostatic belt according to claim 4, characterised in that a rubber-made balloon (5) inflated by fluid is contained in the pocket (3) of said strip (1).

10. A compressive hemostatic belt according to claim 8, characterised in that said confronting surface of the pocket (3) of said strip (1) against the stopping area is formed of an elastic fiber cloth.

11. A compressive hemostatic belt according to claim 9 or claim 10 characterised in that a fluid feed pump (8) and a pressure gauge (9) are connected detachably to the rubber-made balloon (5) by way of a check valve (6).

## Patentansprüche

1. Hämostatischer Druckgürtel, umfassend einen Ballon (5), der durch Füllen mit Fluid aufblasbar ist und durch Befestigungsmittel an einer spezifischen Stelle eines nichtelastischen oder geringelastischen Bandes (1) befestigt ist, gekennzeichnet dadurch, daß ein nichtelastisches Verstärkungselement (11) auf einer Seite des Ballons (5), die dem Band (1) gegenüberliegt, integral aufgebracht ist.

2. Hämostatischer Druckgürtel, umfassend einen Ballon (5), der durch Füllen mit Fluid aufblasbar ist und durch Befestigungsmittel an einer spezifischen Stelle eines nichtelastischen oder geringelastischen Bandes (1) befestigt ist, gekennzeichnet dadurch, daß der Ballon (5) auf der dem Band (1) gegenüberliegenden Seite dick und auf der anderen, dem Stillbereich gegenüberliegenden Seite dünn ist.

3. Hämostatischer Druckgürtel nach Anspruch 1, gekennzeichnet dadurch, daß das Band aus einem nichtelastischen oder geringelastischen Faserstoff hergestellt ist.

4. Hämostatischer Druckgürtel nach einem der Ansprüche 1 bis 3, gekennzeichnet dadurch, daß das Befestigungsmittel eine Tasche (3) ist, die an einer spezifischen Stelle des Bandes (1) ausgebildet ist und den Ballon (5) in sich aufnehmen kann.

5. Hämostatischer Druckgürtel nach Anspruch 4, gekennzeichnet dadurch, daß eine Seite der Tasche (3), die dem Bereich gegenüberliegt, in dem das Bluten gestillt werden soll, aus einem aufweitbaren Streifenelement hergestellt ist.

6. Hämostatischer Druckgürtel nach einem der Ansprüche 1 bis 5, gekennzeichnet dadurch, daß eine Fluidzuführungspumpe (8) und ein Manometer (9) mit Hilfe eines Rückschlagventiles (6) lösbar an den Ballon (5) angeschlossen sind.

7. Hämostatischer Druckgürtel nach Anspruch 1 oder 2, gekennzeichnet dadurch, daß ein Bereich des Bandes (1), der mindestens dem Ballon (5) gegenüberliegt, aus einem nichtelastischen Verstärkungselement gebildet ist, das aus einem Material hergestellt ist, das fester als jenes der anderen Teile ist.

8. Hämostatischer Druckgürtel nach Anspruch 1 oder 2, gekennzeichnet dadurch, daß eine Vielzahl nichtelastischer Verstärkungsfaserstoffe mindestens in dem Bereich des Bandes (1), der dem Ballon (5) gegenüberliegt, übereinander geschichtet sind.

9. Hämostatischer Druckgürtel nach Anspruch 4, gekennzeichnet dadurch, daß ein durch Fluid aufgeblasener, aus Gummi hergestellter Ballon (5) in der Tasche (3) des Bandes (1) aufgenommen ist.

10. Hämostatischer Druckgürtel nach Anspruch 8, gekennzeichnet dadurch, daß die zum Stillbereich gegenüberliegende Fläche der Tasche (3) des Bandes (1) aus einem elastischen Faserstoff gebildet ist.

11. Hämostatischer Druckgürtel nach Anspruch 9 oder 10, gekennzeichnet dadurch, daß eine Fluidzuführungspumpe (8) und ein Manometer (9) mit Hilfe eines Rückschlagventiles (6) lösbar an dem aus Gummi hergestellten Ballon (5) angeschlossen sind.

## Revendications

1. Ceinture hémostatique compressive comprenant un ballonnet (5) pouvant être gonflé en étant rempli d'un fluide, et fixée par des moyens de fixation sur une position spécifique d'une bande (1) non élastique ou à faible élasticité, caractérisée en ce qu'un organe de renforcement non élastique (11) est entièrement appliqué sur un côté dudit ballonnet (5) situé en regard de ladite bande (1).

2. Ceinture hémostatique compressive comprenant un ballonnet (5) pouvant être gonflé en étant rempli d'un fluide, et fixée par des moyens de fixation sur une position spécifique d'une bande non élastique ou à faible élasticité (1), caractérisée en ce que le ballonnet (5) est épais du côté situé en regard de ladite bande (1), et mince de l'autre côté situé en regard de la zone d'arrêt.

3. Ceinture hémostatique compressive selon la revendication 1, caractérisée en ce que ladite bande est constituée d'un tissu de fibres non élastique ou à faible élasticité.

4. Ceinture hémostatique compressive selon l'une quelconque des revendications 1 à 3, caractérisée en ce que ledit moyen de fixation est une poche (3) formée au niveau d'une position spécifique de ladite bande (1), à l'intérieur de laquelle le ballonnet (5) peut être intégré.

5. Ceinture hémostatique compressive selon la revendication 4, caractérisée en ce qu'un côté de ladite poche (3) situé en regard de la zone où le saignement doit être arrêté se compose d'un organe formant feuille dilatable.

6. Ceinture hémostatique compressive selon l'une quelconque des revendications 1 à 5, caractérisée en ce qu'une pompe d'alimentation en fluide (8) et un manomètre (9) sont reliés, de manière détachable, audit ballonnet (5) au moyen d'un clapet de non-retour (6).

7. Ceinture hémostatique compressive selon la revendication 1 ou la revendication 2, caractérisée en qu'une partie de ladite bande (1) située au moins en regard du ballonnet (5) est constituée d'un organe de renforcement non élastique en un matériau plus résistant que celui des autres parties.

8. Ceinture hémostatique compressive selon la revendication 1 ou la revendication 2, caractérisée en ce que plusieurs tissus fibres de renforcement non élastiques sont superposés au moins dans la partie de ladite bande (1) située en regard du ballonnet (5).

9. Ceinture hémostatique compressive selon la revendication 4, caractérisée en ce qu'un ballonnet en caoutchouc (5) gonflé par un fluide est contenu dans la poche (3) de ladite bande (1).

10. Ceinture hémostatique compressive suivant la revendication 8, caractérisée en ce que ladite surface en regard de la poche (3) de ladite bande (1) contre la zone d'arrêt est formée d'un tissu de fibres élastiques.

11. Ceinture hémostatique compressive selon la revendication 9 ou la revendication 10, caractérisée en ce qu'une pompe d'alimentation en fluide (8) et un manomètre (9) sont reliés de manière détachable au ballonnet de caoutchouc (5) au moyen d'un clapet de non retour (6).
